(19) Europäisches Patentamt
European Patent Office
Office européen des brevets

(11) EP 3 727 324 B1

(12) **EUROPEAN PATENT SPECIFICATION**

(45) Date of publication and mention of the grant of the patent:
**26.06.2024 Bulletin 2024/26**

(21) Application number: **18842502.9**

(22) Date of filing: **20.12.2018**

(51) International Patent Classification (IPC):
***A61K 8/39*** (2006.01) ***A61K 8/898*** (2006.01)
***A61K 8/06*** (2006.01) ***A61Q 5/12*** (2006.01)
***A61K 8/04*** (2006.01)

(52) Cooperative Patent Classification (CPC):
**A61K 8/898; A61K 8/046; A61K 8/062; A61K 8/39; A61Q 5/12;** A61K 2800/21; A61K 2800/262

(86) International application number:
**PCT/US2018/066697**

(87) International publication number:
**WO 2019/126442 (27.06.2019 Gazette 2019/26)**

(54) **LOW VISCOSITY CONDITIONER COMPOSITION CONTAINING SILICONE POLYMERS**

KONDITIONIERENDE ZUBEREITUNG MIT NIEDRIGEM VISKOSITÄT ENTHALTEND SILIKONPOLYMERE

COMPOSITION CONDITIONANTE À FAIBLE VISCOSITÉ COMPRENANT DES POLYMÈRES DE SILICONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2017 US 201762608190 P**
**08.08.2018 US 201862715949 P**

(43) Date of publication of application:
**28.10.2020 Bulletin 2020/44**

(73) Proprietor: **The Procter & Gamble Company**
**Cincinnati, OH 45202 (US)**

(72) Inventors:
- **GLENN, Robert, Wayne, Jr.**
  **Cincinnati, Ohio 45202 (US)**
- **YANG, Tiffany, Tien-Yun**
  **Cincinnati, Ohio 45202 (US)**
- **SNYDER, Michael, Albert**
  **Mason, Ohio 45040 (US)**
- **WAGNER, Roland**
  **51373 Leverkusen (DE)**
- **SCHNERING, Albert**
  **51373 Leverkusen (DE)**
- **STREICHER, Katharina**
  **51373 Leverkusen (DE)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2013/148629** **WO-A1-2014/058887**
**US-A- 6 153 569** **US-A1- 2015 093 420**
**US-A1- 2015 359 728** **US-A1- 2016 310 371**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 727 324 B1

## Description

### FIELD OF THE INVENTION

Described herein is a concentrated conditioner composition to be dispensed as a foam from a foam dispenser, wherein the composition comprises one or more silicone polymers. The silicone polymers is a polyorganosiloxane compound containing one or more quaternary ammonium groups, silicone blocks comprising between about 99 and about 199 siloxane units on average, at least one polyalkylene oxide structural unit; and at least one terminal ester group.

### BACKGROUND OF THE INVENTION

A variety of approaches have been developed to condition the hair. A common method of providing conditioning benefit is through the use of conditioning agents such as cationic surfactants and polymers, high melting point fatty compounds, low melting point oils, silicone compounds, and mixtures thereof. Most of these conditioning agents are known to provide various conditioning benefits, and also to be incorporated into compositions in a wide variety of product forms, including but not limited to creams, gels, emulsions, foams and sprays.

Some consumers desire conditioner compositions in a foam form. Given the low density of the foam, conditioner ingredients such as surfactants and conditioning agents may be present at a higher concentration to deliver conditioning benefits in a reasonable volume of foam. Furthermore, in order to be dispensed as a foam from either an aerosol or pump foamer, it may be desirable for the composition to have a low viscosity, for instance 1 centipoise to about 15,000 centipoise.

For example, US Patent Application Publication No. 2015-0359725 A1 discloses a method of treating the hair by the use of a concentrated hair care composition dispensed as a foam from an aerosol foam dispenser, the composition having a liquid phase viscosity of from about 1 centipoise to about 15,000 centipoise and comprising silicones having a particle size of from about 1 nm to about 500 nm. US Patent Application Publication No. 2015-0359726 A1 discloses a method of treating the hair by the use of a concentrated hair care composition dispensed as a foam from a mechanical foam dispenser, the composition having a liquid phase viscosity of from about 1 centipoise to about 80 centipoise and comprising silicones having a particle size of from about 1 nm to about 100 nm.

It can be desirable to add silicone polymers to low viscosity compact conditioner compositions to improve conditioning. However, some silicone polymers are not stable in these compositions, which causes the composition to phase separate, become turbid, and/or exhibit significant color change. Other silicone polymers do not provide acceptable conditioning, as tested by wet combing and dry combing tests and still other silicone polymers may cause dry hair to have low volume.

US Patent application US-A-2015/093420 discloses a hair conditioning composition to provide smooth feel and comprising pre-emulsified silicone polymer dispersion of quaternary groups, silicone block, polyalkylene oxide and terminal ester and gel matrix. International Patent application WO-A-2013/148629 disclsoes low viscosity polyorganosiloxanes comprising a polyorganosiloxane group, a quaternary ammonium group, a terminal ester groupand their use for the modification of surfaces of substrates.

Therefore, there is a need for a low viscosity compact conditioner composition that contains a silicone polymer where the composition is phase stable and has acceptable conditioning and volume.

### SUMMARY OF THE INVENTION

Described herein is a concentrated conditioner composition, as claimed in claim 1, wherein the concentrated conditioner composition comprises: (i) from about 0.5% to about 25% of one or more silicones, by weight of the concentrated conditioner composition, wherein the particle size of the one or more silicones is from about 1 nm to about 100 nm, and wherein at least one or more silicone is a polyorganosiloxane compound comprising: one or more quaternary ammonium groups; silicone blocks comprising between 105 and 180 siloxane units on average; at least one polyalkylene oxide structural unit; and at least one terminal ester group; (ii) from about 60% to about 90% water, by weight of the concentrated conditioner composition; wherein the concentrated conditioner composition has a liquid phase viscosity of from about 1 centipoise to about 15,000 centipoise; wherein the concentrated conditioner composition is to be dispensed as a foam from a foam dispenser.

### BRIEF DESCRIPTION OF THE DRAWINGS

FIG. 1 is a perspective view of an aerosol dispenser according to the present invention having a plastic outer

container and a bag.

FIG. 2A is an exploded perspective view of the aerosol dispenser of FIG. 1 having a collapsible bag.

FIG. 2B is an exploded perspective view of the aerosol dispenser of FIG. 1 having a dip tube.

DETAILED DESCRIPTION OF THE INVENTION

While the specification concludes with claims particularly pointing out and distinctly claiming the invention, it is believed that the present invention will be better understood from the following description.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of".

As used herein, "mixtures" is meant to include a simple combination of materials and any compounds that may result from their combination.

As used herein, "molecular weight" or "M.Wt." refers to the weight average molecular weight unless otherwise stated. Molecular weight is measured using industry standard method, gel permeation chromatography ("GPC").

As used herein, the terms "include," "includes," and "including," are meant to be non-limiting and are understood to mean "comprise," "comprises," and "comprising," respectively.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Conditioner Composition

**A) Silicones**

The concentrated conditioner composition may comprise from about 0.5% to about 25%, alternatively from about 1% to about 20%, alternatively from about 2% to about 18%, alternatively from about 2% to about 10% of one or more silicones, by weight of the concentrated conditioner composition.

The average particle size of the one or more silicones may be from about 1 nm to about 100 nm, alternatively from about 5 nm to about 100 nm, alternatively from about 10 nm to about 80 nm, alternatively about 10 nm to about 60 nm, alternatively about 10 nm to about 50 nm, and alternatively about 10 nm to about 40 nm. The average particle size of the one or more silicones may be less than 100 nm, alternatively less than 90 nm, alternatively less than 80 nm, alternatively less than 70 nm, alternatively less than 60 nm, alternatively less than 50 nm, alternatively less than 40 nm, and alternatively less than 30 nm. The average particle size of the one or more silicones may be greater than 1 nm, greater than 5 nm, greater than 10 nm, and greater than 20 nm.

The particle size of the one or more silicones may be measured by dynamic light scattering (DLS). A Malvern Zetasizer Nano ZEN3600 system (*www.malvern.com*) using He-Ne laser 633nm may be used used for the measurement at 25°C.

The autocorrelation function may be analyzed using the Zetasizer Software provided by Malvern Instruments, which determines the effective hydrodynamic radius, using the Stokes-Einstein equation:

$$D = \frac{k_B T}{6\pi\eta R}$$

wherein $k_B$ is the Boltzmann Constant, T is the absolute temperature, r} is the viscosity of the medium, D is the mean diffusion coefficient of the scattering species, and R is the hydrodynamic radius of particles.

Particle size (i.e. hydrodynamic radius) may be obtained by correlating the observed speckle pattern that arises due to Brownian motion and solving the Stokes-Einstein equation, which relates the particle size to the measured diffusion constant, as is known in the art.

Polydispersity index (PDI) is a dimensionless measure of the broadness of the size distribution calculated from the cumulants analysis in Dynamic Light Scattering and is calculated according to the following equation.

Polydispersity index (PDI) = (the square of standard deviation) / (the square of mean diameter)

The polydispersity index (PDI) of the average silicone droplet size can be less than 0.5, alternatively less than 0.4, alternatively less than 0.3, and alternatively less than 0.2. The PDI of the average silicone size can be from 0 to about 0.6, alternatively from about 0 to about 0.4, alternatively from about 0 to about 0.2.

For each sample, 3 measurements may be made and Z-average values may be reported as the particle size. Other methods may also be employed to measure particle size included, but not limited to, cryo scanning electron microscopy, cryo transmission electron microscopy and lazer diffraction methods.

The one or more silicones may be in the form of a nanoemulsion. The particle size referred to herein is z-average measured by dynamic light scattering. The nanoemulsion may comprise any silicone suitable for application to the skin and/or hair. From about 25% to about 100% of the one or more silicones can be in the form of a nanoemulsion, in another embodiment from about 50% to about 100% of the one or more silicones can be in the form of a nanoemulsion, and in another embodiment from about 75% to about 100% of the one or more silicones can be in the form of a nanoemulsion.

The one or more silicones can have a silicone deposition after 6 treatment cycles from about 100 ppm to about 4000 ppm, alternatively 200 ppm to about 3000 ppm, alternatively from about 400 ppm to about 2500 ppm. The silicone deposition can be measured by the Silicone Deposition Test Method, described hereafter.

**A. Silicone Polymer Containing Quaternary Groups**

The compositions of the present invention comprise a low viscosity silicone polymer having a viscosity up to 100,000 mPa.s. The silicone polymer can have a viscosity from about 1 cP to about 50,000 cP, alternatively from about 25 cP to about 30,000 cP, alternatively from about 50 cP to about 25,000 cP, alternatively from about 100 cP to about 20,000 cP, alternatively from about 150 cP to about 15,000 cP, alternatively from about 200 cP to about 13,000 cP. The silicone polymer can have a viscosity from about 8,000 cP to about 15,000 cP, alternatively from about 9,000 cP to about 13,000 cP, alternatively from bout 10,000 cP to about 12,000 cP. Without being bound by theory, this low viscosity silicone polymer provides improved conditioning benefits over conventional silicones because of the addition of hydrophilic functionalities - quaternary amines, ethylene oxides/propylene oxides. Compared to previously disclosed silicones with quaternary functionality, these new structures are significantly lower in viscosity, so that they don't have to be blended with other lower viscosity diluents and dispersants to allow them to be formulated into products. Low viscosity silicone solvents and diluents can often cause viscosity and stability tradeoffs in hair care products products. The current invention eliminates the need for these materials since the silicone polymer is low enough in viscosity to be added directly or in emulsion form. The improved conditioning benefits include smooth feel, reduced friction, and prevention of hair damage, while, in some embodiments, eliminating the need for a silicone blend.

Structurally, the silicone polymer is a polyorganosiloxane compound comprising one or more quaternary ammonium groups, at least one silicone block comprising an average between 105 and 180 siloxane units, at least one polyalkylene oxide structural unit, and at least one terminal ester group. The silicone block may comprise from about 110 to about 199 siloxane units, alternatively about 120 to about 199 siloxane units, alternatively about 130 to about 199 siloxane units, alternatively about 110 to about 190 siloxane units, alternatively about 130 to about 190 siloxane units, alternatively about 110 to about 175 siloxane units, alternatively about 120 to about 175 siloxane units, alternatively about 130 to about 175 siloxane units, alternatively about 110 to about 155 siloxane units, alternatively about 120 to about 155 siloxane units, alternatively about 130 to about 155 siloxane units, alternatively about 155 to about 199 siloxane units, alternatively about 155 to about 190 siloxane units, and alternatively about 155 to about 175 siloxane units. The silicone block may comprise on average from about 120 to about 170 siloxane units, and alternatively from about 120 to about 145 siloxane units. The silicone block may comprise on average from about 145 to about 170 siloxane units. The average block length' reflect mean values. They can be determined by i.e. 1H-NMR spectroscopy or GPC using protocols known in the art.

The polyorganosiloxane compounds can have a molar ratio of silicone to alkylene oxide block of about 2:1 to about 20:1, alternatively from about 4:1 to about 16:1, alternatively from about 6:1 to about 12:1, and alternatively from about 8:1 to about 10:1.

The nitrogen content for the polyoranosiloxane compounds can be from about 0.13 to about 0.27 mmol N/g

polymer. The nitrogen content for the polyoranosiloxane compounds can be from about 0.13 to about 0.35 mmol N/g polymer, alternatively from about 0.15 to about 0.3 mmol N/g polymer, alternatively from about 0.17 to about 0.27 mmol N/g polymer, and alternatively from about 0.19 to about 0.24 mmol N/g polymer.

The polyorganosiloxane compounds may have the general formulas (Ia) and (Ib):

$$M\text{-}Y\text{-}[\text{-}(N^+R_2\text{-}T\text{-}N^+R_2)\text{-}Y\text{-}]_{m\text{-}}[\text{-}(NR^2\text{-}A\text{-}E\text{-}A'\text{-}NR^2)\text{-}Y\text{-}]_k\text{-}M \qquad \text{(Ia)}$$

$$M\text{-}Y\text{-}[\text{-}(N^+R_2\text{-}T\text{-}N^+R_2)\text{-}Y\text{-}]_m\text{-}[\text{-}(N^+R^2{}_2\text{-}A\text{-}E\text{-}A'\text{-}N^+R^2{}_2)\text{-}Y\text{-}]_k\text{-}M \qquad \text{(Ib)}$$

wherein:

m is > 0, alternatively 0.01 to 100, alternatively 0.1 to 100, alternatively 1 to 100, alternatively 1 to 50, alternatively 1 to 20, and alternatively 1 to 10,
k is 0 or an average value of from ≥ 0 to 50, alternatively from ≥ 0 to 20, and alternatively from ≥ 0 to 10,
M represents a terminal group, comprising terminal ester groups selected from

$-OC(O)-Z$

$-OS(O)_2-Z$

$-OS(O_2)O-Z$

$-OP(O)(O-Z)OH$

$-OP(O)(O-Z)_2$

wherein Z is selected from monovalent organic residues having up to 40 carbon atoms, optionally comprising one or more hetero atoms;
A and A' each are independently from each other selected from a single bond or a divalent organic group having up to 10 carbon atoms and one or more hetero atoms, and E is a polyalkylene oxide group of the general formula:

$$-[CH_2CH_2O]_q-[CH_2CH(CH_3)O]_r-[CH_2CH(C_2H_5)O]_s-$$

wherein q=0 to 200, alternatively 0 to 100, alternatively 0 to 50, alternatively 0 to 25, alternatively 0 to 10, alternatively 1 to 200, alternatively 1 to 100, alternatively 1 to 50, alternatively 1 to 25, and alternatively 1 to 10;
r=0 to 200, alternatively 0 to 100, alternatively 0 to 50, alternatively 0 to 25, and alternatively 0 to 10;
s=0 to 200, alternatively 0 to 100, alternatively 0 to 50, alternatively 0 to 25, and alternatively 0 to 30;
q+r+s = 1 to 600, or alternatively from 1 to 100, or alternatively from 1 to 50, or alternatively from 1 to 40
with percentage of q in (q/(q+r+s)) = 0%, 0.166% to 100%, 1% to 100%, 2% to 100%, 2.5% to 100%, 10% to 100%, 30% to 100%, 50% to 100%; alternatively percentage of q in (q/(q+r+s)) = at least 1%, alternatively at least 2%, alternatively at least 10%, alternatively at least 30% alternatively at least 50%, alternatively at least 75%, alternatively at least 90%, alternatively at least 95%, and alternatively 100%.

$R^2$ is selected from hydrogen or R,
R is selected from monovalent organic groups having up to 22 carbon atoms and optionally one or more heteroatoms, and wherein the free valencies at the nitrogen atoms are bound to carbon atoms,
Y is a group of the formula:

-K-S-K- and -A-E-A'-or-A'-E-A-,

with S=

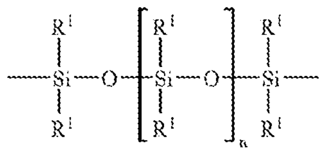

wherein R1 = $C_1$-$C_{22}$-alkyl, $C_1$-$C_{22}$-fluoralkyl or aryl; n=99 to 199 on average, alternatively 110 to 199 on average, alternatively 120 to 199 on average, alternatively 130 to 199 on average, alternatively 110 to 190 on average, alternatively 130 to 190 on average, alternatively 110 to 175 on average, alternatively 120 to 175 on average, alternatively 130 to 175 on average, alternatively 110 to 155 on average, alternatively 120 to 155 on average, alternatively 130 to 155 on average, alternatively 155 to 199 on average, alternatively 155 to 190 on average, alternatively 155 to 175 on average and these can be identical or different if several S Groups are present in the polyorganosiloxane compound;
K is a bivalent or trivalent straight chain, cyclic and/or branched $C_2$-$C_{40}$ hydrocarbon residue which is optionally interrupted by-O-,-NH-, trivalent N, -$NR^1$-,-C(O)-, -C(S)-, and optionally substituted with—OH, wherein $R^1$ is defined as above,

T is selected from a divalent organic group having up to 20 carbon atoms and optionally one or more hetero atoms.

The residues K may be identical or different from each other. In the —K—S—K— moiety, the residue K is bound to the silicon atom of the residue S via a C-Si-bond.
Due to the possible presence of amine groups (-($NR^2$-A-E-A'-$NR^2$)-) in the polyorganosiloxane compounds, they may have protonated ammonium groups, resulting from the protonation of such amine groups with organic or inorganic acids. Such compounds are sometimes referred to as acid addition salts of the polyorganosiloxane compounds according to the invention.
The molar ratio of the quaternary ammonium groups b) and the terminal ester groups c) is less than 20:3, alternatively less than 5:1, alternatively less than 10:3 and alternatively less than 2:1. The ratio can be determined by $^{13}$C-NMR or 1H-NMR.
The silicone polymer has a viscosity at 20 °C and a shear rate of $0.1s^{-1}$ (plate-plate system, plate diameter 40mm, gap width 0.5mm) of less than 100,000 mPa·s (100 mPa·s). The viscosities of the neat silicone polymers may range from about 500 to about 100,000 mPa·s, alternatively from about 500 to about 70,000 mPa·s, alternatively from about 500 to about 50,000 mPa·s, alternatively from about 500 to about 30,000 mPa·s, alternatively from about 2,000 to about 100,000 mPa·s, alternatively from about 2,000 to about 70,000 mPa·s, alternatively from about 2,000 to about 50,000 mPa·s, alternatively from about 2,000 to about 30,000 mPa·s, alternatively from about 8,000 to about 100,000 mPa·s, alternatively from about 8,000 to about 70,000 mPa·s, alternatively from about 8,000 to about 50,000 mPa·s, alternatively from about 8,000 to about 30,000 mPa·s, alternatively from about 15,000 to about 100,000 mPa·s, alternatively from about 15,000 to about 70,000 mPa·s, alternatively from about 15,000 to about 50,000 mPa·s, alternatively from about 15,000 to about 30,000 mPa·s determined at 20 °C and a shear rate of 0.1 $s^{-1}$.
In addition to the above listed silicone polymers, it can be desirably to use the embodiments provided below. For example, in the polyalkylene oxide group E of the general formula:

$$-[CH_2CH_2O]_q-[CH_2CH(CH_3)O]_r-[CH_2CH(C_2H_5)O]_s-$$

wherein the q, r, and s indices may be defined as follows:

q=1 to 200, or preferably from 1 to 100, or more preferably from 1 to 50, or even more preferably from 1 to 20,
r=0 to 200, or preferably from 0 to 100, or more preferably from 0 to 50, or even more preferably from 0 to 20,
s=0 to 200, or preferably from 0 to 100, or more preferably from 0 to 50, or even more preferably from 0 to 20,
q+r+s = 1 to 600, or preferably from 1 to 100, or more preferably from 1 to 50, or even more preferably from 1 to 40 with percentage of q in (q/(q+r+s)) 0%, 0.166% to 100%, 1% to 100%, 2% to 100%, 2.5% to 100%, 10% to 100%, 30% to 100%, 50% to 100%.

For polyorganosiloxane structural units with the general formula S:

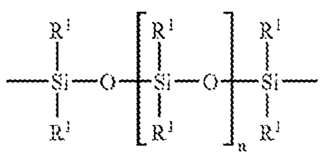

$R^1$=$C_1$-$C_{22}$-alkyl, $C_1$-$C_{22}$-fluoralkyl or aryl; n= from 99 to 199, K (in the group —K—S—K—) is preferably a bivalent or trivalent straight chain, cyclical or branched $C_2$-$C_{20}$ hydrocarbon residue which is optionally interrupted by-O-,-NH-, trivalent N,-$NR^1$-,-C(O)-,-C(S)-, and optionally substituted with—OH.
$R^1$ can be $C_1$-$C_{18}$ alkyl, $C_1$-$C_{18}$ fluoroalkyl and aryl. Furthermore, $R^1$ is preferably $C_1$-$C_{18}$ alkyl, $C_1$-$C_6$ fluoroalkyl

and aryl. Furthermore, $R^1$ is more preferably $C_1$-$C_6$ alkyl, $C_1$-$C_6$ fluoroalkyl, even more preferably $C_1$-$C_4$ fluoroalkyl, and phenyl. Most preferably, $R^1$ is methyl, ethyl, trifluoropropyl and phenyl.

As used herein, the term "$C_1$-$C_{22}$ alkyl" means that the aliphatic hydrocarbon groups possess from 1 to 22 carbon atoms which can be straight chain or branched. Methyl, ethyl, propyl, n-butyl, pentyl, hexyl, heptyl, nonyl, decyl, undecyl, isopropyl, neopentyl and 1,2,3-trimethyl hexyl moieties serve as examples.

Further as used herein, the term "$C_1$-$C_{22}$ fluoroalkyl" means aliphatic hydrocarbon compounds with 1 to 22 carbon atoms which can be straight chain or branched and are substituted with at least one fluorine atom. Monofluormethyl, monofluoroethyl, 1,1,1-trifluorethyl, perfluoroethyl, 1,1,1-trifluoropropyl, 1,2,2-trifluorobutyl are suitable examples.

Moreover, the term "aryl" means unsubstituted or phenyl substituted once or several times with OH, F, Cl, $CF_3$, $C_1$-$C_6$ alkyl, $C_1$-$C_6$ alkoxy, $C_3$-$C_7$ cycloalkyl, $C_2$-$C_6$ alkenyl or phenyl. Aryl may also mean naphthyl.

The positive charges resulting from the ammonium group(s) of the polyorganosiloxane, can be neutralized with inorganic anions such as chloride, bromide, hydrogen sulfate, sulfate, or organic anions, like carboxylates deriving from $C_1$-$C_{30}$ carboxylic acids, for example acetate, propionate, octanoate, especially from $C_{10}$-$C_{18}$ carboxylic acids, for example decanoate, dodecanoate, tetradecanoate, hexadecanoate, octadecanoate and oleate, alkylpolyethercarboxylate, alkylsulphonate, arylsulphonate, alkylarylsulphonate, alkylsulphate, alkylpolyethersulphate, phosphates derived from phosphoric acid mono alkyl/aryl ester and phosphoric acid dialkyl/aryl ester. The properties of the polyorganosiloxane compounds can be, *inter alia,* modified based upon the selection of acids used.

The quaternary ammonium groups are usually generated by reacting the di-tertiary amines with an alkylating agents, selected from in particular di-epoxides (sometimes referred to also as bis-epoxides) in the presence of mono carboxylic acids and difunctional dihalogen alkyl compounds.

The polyorganosiloxane compounds can be of the general formulas (Ia) and (Ib):

$$M\text{-}Y\text{-}[\text{-}(N^+R_2\text{-}T\text{-}N^+R_2)\text{-}Y\text{-}]_m\text{-}[\text{-}(NR^2\text{-}A\text{-}E\text{-}A'\text{-}NR^2)\text{-}Y\text{-}]_k\text{-}M \qquad (Ia)$$

$$M\text{-}Y\text{-}[\text{-}(N^+R_2\text{-}T\text{-}N^+R_2)\text{-}Y\text{-}]_m\text{-}[\text{-}(N^+R^2{}_2\text{-}A\text{-}E\text{-}A'\text{-}N^+R^2{}_2)\text{-}Y\text{-}]_k\text{-}M \qquad (Ib)$$

wherein each group is as defined above; however, the repeating units are in a statistical arrangement (i.e., not a blockwise arrangement).

The polyorganosiloxane compounds may be also of the general formulas (IIa) or (IIb):

$$M\text{-}Y\text{-}[\text{-}N^+R_2\text{-}Y\text{-}]_m\text{-}[\text{-}(NR^2\text{-}A\text{-}E\text{-}A'\text{-}NR^2)\text{-}Y\text{-}]_k\text{-}M \qquad (IIa)$$

$$M\text{-}Y\text{-}[\text{-}N^+R_2\text{-}Y\text{-}]_m\text{-}[\text{-}(N^+R^2{}_2\text{-}A\text{-}E\text{-}A'\text{-}N^+R^2{}_2)\text{-}Y\text{-}]_k\text{-}M \qquad (IIb)$$

wherein each group is as defined above. Also in such formula the repeating units are usually in a statistical arrangement (i.e not a block-wise arrangement).
wherein, as defined above, M is

-OC(O)-Z,

$-OS(O)_2$-Z

$-OS(O_2)$O-Z

-OP(O)(O-Z)OH

$-OP(O)(O\text{-}Z)_2$

Z is a straight chain, cyclic or branched saturated or unsaturated $C_1$-$C_{20}$, or preferably $C_2$ to $C_{18}$, or even more preferably a hydrocarbon radical, which can be interrupted by one or more -O- , or -C(O)- and substituted with -OH. M can be -OC(O)-Z resulting from normal carboxylic acids in particular with more than 10 carbon atoms like for example dodecanoic acid.

The molar ratio of the polyorganosiloxane-containing repeating group -K-S-K-and the polyalkylene repeating group -A-E-A'- or -A'-E-A- is between 1:100 and 100:1, alternatively between 1:20 to 20:1, or alternatively between 1:10 to 10:1.

In the group $-(N^+R_2\text{-}T\text{-}N^+R_2)$-, R may represent a monovalent straight chain, cyclic or branched $C_1$-$C_{20}$ hydro-

carbon radical, which can be interrupted by one or more -O- , - C(O)- and can be substituted by-OH, T may represent a divalent straight-chain, cyclic, or branched $C_1$-$C_{20}$ hydrocarbon radical, which can be interrupted by -O-, -C(O)- and can be substituted by hydroxyl.

The above described polyorganosiloxane compounds comprising quaternary ammonium functions and ester functions may also contain: 1) individual molecules which contain quaternary ammonium functions and no ester functions; 2) molecules which contain quaternary ammonium functions and ester functions; and 3) molecules which contain ester functions and no quaternary ammonium functions. While not limited to structure, the above described polyorganosiloxane compounds comprising quaternary ammonium functions and ester functions are to be understood as mixtures of molecules comprising a certain averaged amount and ratio of both moieties.

Various monofunctional organic acids may be utilized to yield the esters caninclude $C_1$-$C_{30}$ carboxylic acids, for example $C_2$, $C_3$, $C_8$ acids, $C_{10}$-$C_{18}$ carboxylic acids, for example $C_{12}$, $C_{14}$, $C_{16}$ acids, saturated, unsaturated and hydroxyl functionalized $C_{18}$ acids, alkylpolyethercarboxylic acids, alkylsulphonic acids, arylsulphonic acids, alkylarylsulphonic acids, alkylsulphuric acids, alkylpolyethersulphuric acids, phosphoric acid mono alkyl/aryl esters and phosphoric acid dialkyl/aryl esters.

**B) Nonionic Emulsifiers**

The concentrated conditioner composition may comprise from about 1% to about 15%, and alternatively from about 1.5% to about 12% of a nonionic emulsifier, by weight of the concentrated conditioner composition. The one or more silicones may be supplied in the form of a nanoemulsion comprising a nonionic emulsifier. Nonionic emulsifiers are alcohol ethoxylates which are condensation products of aliphatic alcohols having from about 8 to about 18 carbon atoms, in either straight chain or branched chain configuration, with from about 2 to about 35 moles of ethylene oxide, e.g., a coconut alcohol ethylene oxide condensate having from about 2 to about 30 moles of ethylene oxide per mole of coconut alcohol, the coconut alcohol fraction having from about 10 to about 14 carbon atom.

The nonionic emulsifier may have a hydrocarbon chain length of from about 16 to about 20 carbon atoms and from about 20 to about 25 moles of ethoxylate.

The nonionic emulsifier may have a hydrocarbon chain length of from about 19 to about 11 carbon atoms, alternatively from about 9 to about 11 carbon atoms, and from about 2 to about 4 moles of ethoxylate.

The nonionic emulsifier may comprise a combination of (a) a nonionic emulsifier having a hydrocarbon chain that is branched, has a length of from about 11 to about 15 carbon atoms, and has from about 5 to about 9 moles of ethoxylate; and (b) a nonionic emulsifier having a hydrocarbon chain that has a length of from about 11 to about 13 carbon atoms and has from about 9 to about 12 moles of ethoxylate.

The nanoemulsions used in this invention may be prepared by two different methods: (1) mechanical, and (2) emulsion polymerization.

The first method of preparing the nanoemulsion is the mechanical method in which the nanoemulsion is prepared via the following steps: (1) a primary surfactant is dissolved in water, (2) a silicone is added, and a two-phase mixture is formed, (3) with simple mixing, a co-surfactant is slowly added to the two-phase mixture, until a clear isotropic nanoemulsion of a siloxane-in-water is formed.

The second method of preparing the nanoemulsion is by emulsion polymerization. polymer precursors, i.e., monomers, or reactive oligomers, which are immiscible in water; a surfactant to stabilize polymer precursor droplets in water; and a water soluble polymerization catalyst. Typically, the catalyst is a strong mineral acid such as hydrochloric acid, or a strong alkaline catalyst such as sodium hydroxide. These components are added to water, the mixture is stirred, and polymerization is allowed to advance until the reaction is complete, or the desired degree of polymerization (DP) is reached, and an emulsion of the polymer is formed.

The nonionic emulsifier in the formulation has hydrocarbon chain length of 16-20 and 20-25 ethoxylate mole.

The hydrocarbon chain length and the ethoxylate mole number for the nonionic emulsifer is 9-11 and 2-4, respectively.

The nonionic emulsifier system consists of a) C11-15 with 5-9 ethoxylate moles in branched configuration b) linear nonionic emulsifier with C11-13 hydrocarbon chain length and 9-12 moles of ethoxylate.

**C) High Melting Point Fatty Compounds**

The concentrated conditioner composition may comprise from about 0.25% to about 5%, alternatively from about 0.5% to about 4%, and alternatively from about 1% to about 3% of high melting point fatty compound, by weight of the concentrated conditioner composition. The high melting point fatty compounds have a melting point of about 25 °C or higher, and are selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also

be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than about 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The fatty alcohols described herein are those having from about 14 to about 30 carbon atoms, preferably from about 16 to about 22 carbon atoms. These fatty alcohols are saturated and can be straight or branched chain alcohols. Nonlimiting examples of fatty alcohols include cetyl alcohol, stearyl alcohol, behenyl alcohol, and mixtures thereof.

The fatty acids useful herein are those having from about 10 to about 30 carbon atoms, preferably from about 12 to about 22 carbon atoms, and more preferably from about 16 to about 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through steareth- 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, i.e., a mixture of fatty alcohols containing predominantly cetyl and stearyl alcohol, wherein the numeric designation indicates the number of ethylene glycol moieties present; C16 -C30 alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

The fatty compound may be a single high melting point compound of high purity. Single compounds of pure fatty alcohols selected may be selected from the group consisting of pure cetyl alcohol, stearyl alcohol, and behenyl alcohol. By "pure" herein, what is meant is that the compound has a purity of at least about 90%, alternatively at least about 95%.

Commercially available high melting point fatty compounds described herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago, Illinois USA), HYSTRENE available from Witco Corp. (Dublin, Ohio USA), and DERMA available from Vevy (Genova, Italy).

### D) Cationic Surfactants

The concentrated conditioner composition may comprise from about 0.25% to about 5%, alternatively from about 0.5% to about 4%, and alternatively from about 1% to about 3% cationic surfactants, by weight of the concentrated conditioner composition.

The cationic surfactant may be a mono-long alkyl quaternized ammonium salt having the formula (XIII) [from WO2013148778]:

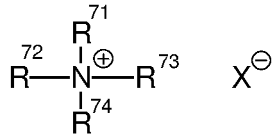

(XIII)

wherein one of $R^{71}$ , $R^{72}$ $R^{73}$ a n $R^{74}$ selected from an aliphatic group of from about 14 to about 30 carbon atoms or an aromatic, alkoxy, polyoxyalkylene, alkylamido, hydroxyalkyl, aryl or alkylaryl group

having up to about 30 carbon atoms; the remainder of $R^{71}$, $R^{72}$ $R^{73}$ and $R^{74}$ are independently selected from an aliphatic group of from about 1 to about 8 carbon atoms or an aromatic, alkoxy, polyoxyalkylene, alkylamido, hydroxyalkyl, aryl or alkylaryl group having up to about 8 carbon atoms; and X is a salt-forming anion such as those selected from halogen, (e.g., chloride, bromide), acetate, citrate, lactate, glycolate, phosphate, nitrate, sulfonate, sulfate, alkylsulfate, glutamate, and alkyl sulfonate radicals. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, one of $R^{71}$ , $R^{72}$ $R^{73}$ and $R^{74}$ is selected from an alkyl group of from about 14 to about 30 carbon atoms, more preferably from about 16 to about 22 carbon atoms, still more preferably from about 16 to about 18 carbon atoms; the remainder of $R^{71}$, $R^{72}$ , $R^{73}$, and $R^{74}$ are independently selected from the group consisting of $CH_3$, $C_2H_5$, $C_2H_4OH$, $CH_2C_5H_5$, and mixtures thereof; and (X) is selected from the group consisting of Cl, Br, $CH_3OSO_3$, and mixtures thereof. It is believed that such mono-long alkyl quatemized ammonium salts can provide improved slippery and slick feel on wet hair.

Nonlimiting examples of such mono-long alkyl quatemized ammonium salt cationic surfactants include: behenyl trimethyl ammonium chloride available, for example, with tradename Genamine KDMP from Clariant, with tradename INCROQUAT TMC-80 from Croda and ECONOL TM22 from Sanyo Kasei; stearyl trimethyl ammonium chloride available, for example, with tradename CA-2450 from Nikko Chemicals; cetyl trimethyl ammonium chloride available, for example, with tradename CA-2350 from Nikko Chemicals; behenyltrimethylammonium methyl sulfate, available from FeiXiang; hydrogenated tallow alkyl trimethyl ammonium chloride; stearyl dimethyl benzyl ammonium chloride; and stearoyl amidopropyl dimethyl benzyl ammonium chloride.

Among them, more preferred cationic surfactants are those having a shorter alkyl group, i.e., $C_{16}$ alkyl group. Such cationic surfactant includes, for example, cetyl trimethyl ammonim chloride. It is believed that cationic surfactants having a shorter alkyl group are advantageous for concentrated conditioner silicone nanoemulsion compositions of the present invention comprising a cationic surfactant and with improved shelf stability.

**E) Water Miscible Solvents**

The concentrated conditioner compositions described herein may comprise from about 0.01% to about 25%, alternatively from about 0.01% to about 20%, and alternatively from about 0.01% to about 15% of a water miscible solvent, by weight of the concentrated conditioner composition. The concentrated conditioner compositions described herein may comprise 0% of a water miscible solvent, by weight of the concentrated conditioner composition. Non-limiting examples of suitable water miscible solvents include polyols, copolyols, polycarboxylic acids, polyesters and alcohols.

Examples of useful polyols include, but are not limited to, glycerin, diglycerin, propylene glycol, ethylene glycol, butylene glycol, pentylene glycol, 1,3-butylene glycol, cyclohexane dimethanol, hexane diol, polyethylene glycol (200-600), sugar alcohols such as sorbitol, manitol, lactitol and other mono- and polyhydric low molecular weight alcohols (e.g., $C_2$-$C_8$ alcohols); mono di- and oligo-saccharides such as fructose, glucose, sucrose, maltose, lactose, and high fructose corn syrup solids and ascorbic acid.

Examples of polycarboxylic acids include, but are not limited to citric acid, maleic acid, succinic acid, polyacrylic acid, and polymaleic acid.

Examples of suitable polyesters include, but are not limited to, glycerol triacetate, acetylated-monoglyceride, diethyl phthalate, triethyl citrate, tributyl citrate, acetyl triethyl citrate, acetyl tributyl citrate.

Examples of suitable dimethicone copolyols include, but are not limited to, PEG-12 dimethicone, PEG/PPG-18/18 dimethicone, and PPG-12 dimethicone.

Examples of suitable alcohols include, but are not limited to ethanol, n-propanol, isopropanol, n-butanol, sec-butanol, tert-butanol, n-hexanol and cyclohexanol.

Other suitable water miscible solvents include, but are not limited to, alkyl and allyl phthalates; napthalates; lactates (e.g., sodium, ammonium and potassium salts); sorbeth-30; urea; lactic acid; sodium pyrrolidone carboxylic acid (PCA); sodium hyraluronate or hyaluronic acid; soluble collagen; modified protein; monosodium L-glutamate; alpha & beta hydroxyl acids such as glycolic acid, lactic acid, citric acid, maleic acid and salicylic acid; glyceryl polymethacrylate; polymeric plasticizers such as polyquaterniums; proteins and amino acids such as glutamic acid, aspartic acid, and lysine; hydrogen starch hydrolysates; other low molecular weight esters (e.g., esters of $C_2$-$C_{10}$ alcohols and acids); and any other water soluble plasticizer known to one skilled in the art of the foods and plastics industries; and mixtures thereof.

The water miscible solvents may be selected from the group consisting of glycerin, propylene glycol, dipropylene glycol, and mixtures thereof. EP 0283165 B1 discloses other suitable water miscible solvents, including glycerol derivatives such as propoxylated glycerol.

### F) Viscosity Modifiers

The concentrated conditioner composition described herein may comprise from about 0.001% to about 2%, alternatively from about 0.001% to about 1%, and alternatively from about 0.001% to about 0.5% of a viscosity modifier, by weight of the concentrated conditioner composition. The concentrated conditioner composition described herein may comprise 0% of a viscosity modifier, by weight of the concentrated conditioner composition. Non-limiting examples of suitable viscosity modifiers include water soluble polymers, cationic water soluble polymers,

Examples of water soluble polymers include, but are not limited to (1) vegetable based polymers such as gum Arabic, tragacanth gum, galactan, guar gum, carob gum, karaya gum, carrageenan, pectin, agar, quince seed, algal colloid, starch (rice, corn, potato, or wheat), and glycyrrhizinic acid; (2) microorganism-based polymers such as xanthan gum, dextran, succinoglucan, and pullulan; and (3) animal-based polymers such as collagen, casein, albumin, and gelatin. Examples of semi-synthetic water-soluble polymers include (1) starch-based polymers such as carboxymethyl starch and methylhydroxypropyl starch; (2) cellulose-based polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose (CMC), crystalline cellulose, and cellulose powder; and (3) alginate-based polymers such as sodium alginate and propylene glycol alginate. Examples of synthetic water-soluble polymers include (1) vinyl-based polymers such as polyvinyl alcohol, polyvinyl methyl ether- based polymer, polyvinylpyrrolidone, and carboxyvinyl polymer (CARBOPOL 940, CARBOPOL 941; (2) polyoxyethylene-based polymers such as polyethylene glycol 20,000, polyethylene glycol 6,000, and polyethylene glycol 4,000; (3) copolymer-based polymers such as a copolymer of polyoxyethylene and polyoxypropylene, and PEG/PPG methyl ether; (4) acryl-based polymers such as poly(sodium acrylate), poly(ethyl acrylate), polyacrylamide, polyethylene imines, and cationic polymers. The water-swellable clay minerals are nonionic water-soluble polymers and correspond to one type of colloid-containing aluminum silicate having a triple layer structure. More particular, as examples thereof, mention may be made of bentonite, montmorillonite, beidellite, nontronite, saponite, hectorite, aluminum magnesium silicate, and silicic anhydride.

Examples of cationic water soluble polymers include, but are not limited to (1) quaternary nitrogen-modified polysaccharides such as cation-modified cellulose, cation-modified hydroxyethylcellulose, cation-modified guar gum, cation-modified locust bean gum, and cation-modified starch; (2) dimethyldiallylammonium chloride derivatives such as a copolymer of dimethyldiallylammonium chloride and acrylamide, and poly(dimethylmethylene piperidinium chloride); (3) vinylpyrrolidone derivatives such as a salt of a copolymer of vinylpyrrolidone and dimethylaminoethyl methacrylic acid, a copolymer of vinylpyrrolidone and methacrylamide propyltrimethylammonium chloride, and a copolymer of vinylpyrrolidone and methylvinylimidazolium chloride; and (4) methacrylic acid derivatives such as a copolymer of methacryloylethyldimethylbetaine, methacryloylethyl trimethylammonium chloride and 2-hydroxyethyl methacrylate, a copolymer of methacryloylethyldimethylbetaine, and methacryloylethyl trimethylammonium chloride and methoxy polyethylene glycol methacrylate.

### G) Viscosity

The concentrated hair care composition described herein may have a liquid phase viscosity of from about 1 centipoise to about 2,500 centipoise, alternatively from about 5 centipoise to about 2,000 centipoise, alternatively from about 10 centipoise to about 1,500 centipoise, and alternatively from about 15 centipoise to about 1,000 centipoise. In an embodiment, the concentrated hair care composition described herein may have a liquid phase viscosity of from about 1 centipoise to about 15,000 centipoise, alternatively from about 1 centipoise to about 8,000 centipoise, alternatively from about 5 centipoise to about 5,000 centipoise, alternatively from about 10 centipoise to about 2,500 centipoise, alternatively from about 15 centipoise to about 1,500 centipoise, and alternatively from about 20 centipoise to about 1,000 centipoise. In an embodiment, the concentrated hair care composition described herein may have a liquid phase viscosity of from about 200 centipoise to about 15,000 centipoise, alternatively from about 300 centipoise to about 12,000 centipoise, alternatively from about 400 centipoise to about 8,000 centipoise, alternatively from about 500 centipoise to about 5,000 centipoise, and alternatively from about 600 centipoise to about 2,500 centipoise, and alternatively from about 700 centipoise to about 2,000 centipoise.

The viscosity values may be measured employing any suitable rheometer or viscometer at 25.0 °C and at a shear rate of about 2 reciprocal seconds. The viscosities reported herein were measured a Cone/Plate Controlled Stress Brookfield Rheometer R/S Plus, by Brookfield Engineering Laboratories, Stoughton, MA. The cone used (Spindle C-75-1) has a diameter of 75 mm and 1° angle. The viscosity is determined using a steady state flow experiment at constant shear rate of 2 $s^{-1}$ and at temperature of 25.0 °C. The sample size is 2.5ml and the total measurement reading time is 3 minutes. The liquid phase viscosity may be measured under ambient conditions and prior to the addition of the propellant.

Especially when the concentrated conditioner composition is dispensed from a mechanical foam dispenser, the concentrated conditioner composition described herein may have a liquid phase viscosity of from about 1 centipoise to about 80 centipoise, alternatively from about 3 to about 60 centipoise, alternatively from about 5 to about 45 centipoise,

and alternatively from about 10 to about 40 centipoise. In one embodiment, the concentrated conditioner composition described herein may have a liquid phase viscosity of from about 1 centipoise to about 150 centipoise, alternatively from about 2 centipoise to about 100 centipoise, alternatively from about 3 centipoise to about 60 centipoise, alternatively from about 5 centipoise to about 45 centipoise, and alternatively from about 10 centipoise to about 40 centipoise.

The viscosity values may be measured employing any suitable rheometer or viscometer at 25.0 °C and at a shear rate of about 2 reciprocal seconds. The viscosity values reported herein were measured using a TA Instruments AR-G2 Rheometer with a concentric cylinder attachment (cup with a diameter of 30.41mm; a bob with a diameter of 27.98 mm and a length of 42.02mm; and a concentric cyclinder jacket assembly) at a shear rate of 2 reciprocal seconds at 25°C.

### H) Optional Ingredients

The concentrated conditioner composition described herein may optionally comprise one or more additional components known for use in hair care or personal care products, provided that the additional components are physically and chemically compatible with the essential components described herein, or do not otherwise unduly impair product stability, aesthetics or performance. Such optional ingredients are most typically those materials approved for use in cosmetics and that are described in reference books such as the CTFA Cosmetic Ingredient Handbook, Second Edition, The Cosmetic, Toiletries, and Fragrance Association, Inc. 1988, 1992. Individual concentrations of such additional components may range from about 0.001 wt% to about 10 wt% by weight of the conditioning composition.

Emulsifiers suitable as an optional ingredient herein include mono- and di-glycerides, fatty alcohols, polyglycerol esters, propylene glycol esters, sorbitan esters and other emulsifiers known or otherwise commonly used to stabilized air interfaces, as for example those used during preparation of aerated foodstuffs such as cakes and other baked goods and confectionary products, or the stabilization of cosmetics such as hair mousses.

Further non-limiting examples of such optional ingredients include preservatives, perfumes or fragrances, cationic polymers, viscosity modifiers, coloring agents or dyes, conditioning agents, hair bleaching agents, thickeners, moisturizers, foam boosters, additional surfactants or nonionic cosurfactants, emollients, pharmaceutical actives, vitamins or nutrients, sunscreens, deodorants, sensates, plant extracts, nutrients, astringents, cosmetic particles, absorbent particles, adhesive particles, hair fixatives, fibers, reactive agents, skin lightening agents, skin tanning agents, antidandruff agents, perfumes, exfoliating agents, acids, bases, humectants, enzymes, suspending agents, pH modifiers, hair colorants, hair perming agents, pigment particles, anti-acne agents, antimicrobial agents, sunscreens, tanning agents, exfoliation particles, hair growth or restorer agents, insect repellents, shaving lotion agents, non-volatile solvents or diluents (water-soluble and water-insoluble), co-solvents or other additional solvents, and similar other materials.

The conditioner composition may comprise from about 0.5% to about 7%, alternatively from about 1% to about 6%, and alternatively from about 2% to about 5% perfume, by weight of the conditioner composition.

The conditioner composition may have a silicone to perfume ratio of from 1:1 to about 19:1, alternatively from about 3:2 to about 9:1, alternatively from about 7:3 to about 17:3.

Examples of suitable perfumes may be provided in the CTFA (Cosmetic, Toiletry and Fragrance Association) 1992 International Buyers Guide, published by CFTA Publications and OPD 1993 Chemicals Buyers Directory 80th Annual Edition, published by Schnell Publishing Co. A plurality of perfume components may be present in the conditioner composition.

### I) Foam Dispenser

Referring to FIGS. 1, 2A, and 2B, an aerosol dispenser 20 is shown. The dispenser 20 comprises a pressurizeable outer container 22. The outer container 22 can comprise any suitable material, including plastic or metal. The outer container 22 may have an opening. The opening defines a neck 24, to which other components may be sealed. The neck 24 may be connected to the container sidewall by a shoulder 25.

Referring to FIGS. 2A and 2B, a valve cup 26 may be sealed to the opening of the outer container 22. The seal, outer container and other container components can be selected to be resistant to the shampoo composition 42 and/or propellant 40.

A valve assembly 28, in turn, may be disposed within the valve cup 26. The valve assembly 28 provides for retention of shampoo composition 42 within the aerosol dispenser 20 until the shampoo composition 42 is selectively dispensed by a user. The valve assembly 28 may be selectively actuated by an actuator 30. Selective actuation of the valve assembly 28 allows the user to dispense a desired quantity of the shampoo composition 42 on demand. The shampoo composition can be dispensed as a foam.

Inside the outer container 22 may be a product delivery device. The product delivery device may comprise a collapsible bag 32 which can be made of gas impermeable material as shown in FIG. 2A. The collapsible bag 32 may be mounted in a sealing relationship to the neck 24 of the container (i.e. a bag-on-can arrangement). Alternative the

collapsible bag 32 may be mounted in sealing relationship to the valve assembly 28 (i.e. a bag-on-valve arrangement).

The collapsible bag 32 may hold shampoo composition 42 therein, and prevent intermixing of such shampoo composition 42 with propellant 40, which can also be referred to as driving gas. The propellant 40 may be stored outside the collapsible bag 32, and inside the outer container 22. The propellant may be any gas as long as it does not excessively penetrate the walls of the collapsible bag 32 or outer container 22 thus maintaining the performance of the product and dispensing acceptable during its usable life.

The shampoo composition 42 may include a propellant, which can also be referred to as a foaming or blooming agent. If a blooming agent is used with the composition 42, the pressure in the outer container 22 can be greater than the vapor pressure of the blooming agent, so that shampoo composition 42 may be dispensed from within the bag.

After the collapsible bag has been filled with the composition, the outer container may be pressurized from about 40 to about 160 psig, from about 50 to about 140 psig, from about 60 to about 90 psig (all measured at RT). In any case, the equilibrium pressure measured at a certain temperature cannot exceed the maximum allowable pressure of the container per the applicable local transport and safety regulations.

The product delivery device may alternatively or additionally comprise a dip tube 34 as shown in FIG. 2B. The dip tube 34 extends from a proximal end sealed to the valve assembly 28. The dip tube 34 may terminate at a distal end juxtaposed with the bottom of the outer container 22. The shampoo composition 42 and propellant 40 can intermix. The propellant 40 also accomplish the function of blooming agent. Both are co-dispensed in response to selective actuation of the valve assembly 28 by a user.

The product delivery device may be an aerosol pump dispenser and may not contain a dip tube or a collapsible bag, for instance, an inverted aerosol container.

The pressure of the propellant 40 within the outer container 22 provides for dispensing of the shampoo composition 42/co-dispensing of shampoo composition 42/propellant 40 to ambient, and optionally to a target surface. The target surface may include a surface to be cleaned or treated by the shampoo composition 42, hair, scalp, etc. Such dispensing occurs in response to the user actuating the valve assembly 28.

The outer container may be pressurized from about 20 to about 110 psig, more preferably from about 30 to about 90 psig, still more preferably from about 40 to about 70 psig (all measured after filling to the intended level at RT). In any case, the equilibrium pressure measured at a certain temperature cannot exceed the maximum allowable pressure of the container per the applicable local transport and safety regulations.

Referring to FIGS. 2A and 2B, the aerosol dispensers 20, and components thereof, may have a longitudinal axis, and may be axi-symmetric and can have a round cross section. Alternatively, the outer container 22, may be eccentric and may have a square, elliptical or other cross section. The outer container 22 and aerosol dispenser 20 may be nonrefillable and may be permanently sealed to prevent reuse without destruction and/or gross deformation of the aerosol dispenser 20. If desired, the outer container 22, collapsible bag 32, and/or dip tube 34, may be transparent or substantially transparent. If the outer container 22 and collapsible bag 32 (if present) are transparent, this arrangement can provide the benefit that the consumer knows when shampoo composition 42 is nearing depletion and allows improved communication of shampoo composition 42 attributes, such as color, viscosity, stability, etc. Alternatively or additionally, the outer container 22 and/or collapsible bag 32, etc. may be transparent and colored with like or different colors.

Alternatively, the hair composition can be stored and dispensed from a mechanical foam dispenser. Non-limiting examples of suitable pump dispensers include those described in WO 2004/078903, WO 2004/078901, and WO 2005/078063 and may be supplied by Albea (60 Electric Ave., Thomaston, CT 06787 USA) or Rieke Packaging Systems (500 West Seventh St., Auburn, Indiana 46706). The composition can be substantialy free of propellant.

Alternatively, the composition can be stored and dispensed from a squeeze foam dispenser. An example of squeeze foamer is EZ'R available from Albéa.

**J) Propellant**

A propellant can be added to the conditioner composition described herein at a conditioner composition to propellant weight ratio of from about 3:17 to about 49:1; alternatively from about 9:1 to about 97:3; and alternatively from about 23:2 to about 24:1 to create a pressurized conditioner composition.

The pressurized conditioner composition can comprise from about 1% to about 12% propellant, alternatively from about 2% to about 10% propellant, alternatively from about 3% to about 8% propellant, alternatively from about 4% to about 6% propellant, from about 1% to about 6% propellant, alternatively from about 2% to about 5% propellant, and alternatively from about 3% to about 4% propellant, by weight of the pressurized conditioner composition.

The pressurized conditioner composition can be dispensed as a foam wherein the foam has a density of from about 0.025 g/cm$^3$ to about 0.30 g/cm$^3$, alternatively from about 0.035 g/cm$^3$ to about 0.20 g/cm$^3$, alternatively from about 0.045 g/cm$^3$ to about 0.15 g/cm$^3$, and alternatively from about 0.055 g/cm$^3$ to about 0.12 g/cm$^3$. The pressurized conditioner composition can be dispensed as a foam wherein the foam as a density of from about 0.025 g/cm$^3$ to about 0.40 g/cm$^3$, alternatively from about 0.035 g/cm$^3$ to about 0.30 g/cm$^3$, alternatively from about 0.025 g/cm$^3$ to about 0.20

g/cm$^3$, alternatively from about 0.045 g/cm$^3$ to about 0.20 g/cm$^3$, alternatively from about 0.045 g/cm$^3$ to about 0.15 g/cm$^3$, alternatively from about 0.055 g/cm$^3$ to about 0.15 g/cm$^3$, and alternatively from about 0.075 g/cm$^3$ to about 0.15 g/cm$^3$.

The propellant may comprise one or more volatile materials, which in a gaseous state, may carry the other components of the pressurized conditioner composition in particulate or droplet form. The propellant may have a boiling point within the range of from about -45° C. to about 5° C. The propellant may be liquefied when packaged in convention aerosol containers under pressure. The rapid boiling of the propellant upon leaving the aerosol foam dispenser may aid in the atomization of the other components of the pressurized conditioner composition.

The propellant which may be employed in the conditioner compositions described herein can include the chemically-inert hydrocarbons such as propane, n-butane, isobutane, n-pentane, isopentane, and mixtures thereof; chlorofluorocarbons (CFCs) such as 20 dichlorodifluoromethane, 1,1-dichloro-1,1,2,2-tetrafluoroethane, 1-chloro-1,1-difluoro-2,2-trifluoroethane, 1-chloro-1,1-difluoroethylene, monochlorodifluoromethane and mixtures thereof; hydrofluorocarbons (HFCs) such as 1,1-difluoroethane, 1,3,3,3-tetrafluoropropene, 2,3,3,3-tetrafluoropropene and mixtures thereof; hydrofluoroolefins (HFOs) such as 2,3,3,3-tetrafluoropropene (HFO-1234yf), trans-1,3,3,3-tetrafluoroprop-1-ene (HFO-1234ze), and mixtures 25 thereof; alkyl ethers such as dimethyl ether, methyl ethyl ether, and mixtures thereof; compressed gases such as carbon dioxide, nitrous oxide, nitrogen, compressed air, and mixtures thereof; and mixtures of one or more hydrocarbons, chlorofluorocarbons, hydrofluorocarbons, hydrofluoroolefins, alkyl ethers, and compressed gases. The propellant can be trans-1,3,3,3-tetrafluoroprop-1 -ene.

The propellant can also comprise a blend of hydrocarbons such as isobutane, 30 propane, and butane including, but not limited to, hydrocarbon blend A-46 (15.2% propane, 84.8% isobutane), hydrocarbon blend NP-46 (25.9% propane, 74.1% n-butane), hydrocarbon blend NIP-46 (21.9% propane, 31.3% isobutane, 46.8% n-butane), and other non-limiting hydrocarbon blends designated as A-31, NP-31, NIP-31, A-70, NP-70, NIP-70, A-85, NP-85, A-108. The propellant may include chlorofluorocarbons (CFCs) including, but not limited to 1,1-dichloroethane (HFC-152a). The propellant may include hydrofluorocarbons (HFCs) including, but not limited to, 1,3,3,3-tetrafluoropropene (HFC-134a). The propellant may include hydrofluoroolefins (HFOs) including, but not limited to, 2,3,3,3-5 tetrafluoropropene (HFO-1234yf), and 1,3,3,3-tetrafluoropropene (HFO-1234ze). The propellant can include compressed gases including, but not limited to, carbon dioxide and nitrous oxide.

**L. Water**

The concentrated conditioner composition described herein may comprise from about from about 60% to about 90% water, alternatively from about 65% to about 87.5%, alternatively from about 67.5% to about 85%, alternatively from about 70% to about 82.5%, and alternatively from about 72.5% to about 80% water.

Method of Treating Hair

The method of conditioning the hair described herein comprises (1) providing a conditioner composition as described herein; (2) adding a propellant to the conditioner composition to create a pressurized conditioner composition; (2) dispensing the pressurized conditioner composition from an aerosol foam dispenser as a dosage of foam; (3) applying the foam to the hair; and (4) rinsing the foam from the hair.

Alternatively, the method of treating the hair described herein comprises (1) providing a concentrated conditioner composition, as described herein, in a mechanical foam dispenser, (2) dispensing the concentrated conditioner composition from the mechanical foam dispenser as a dosage of foam; (3) applying the foam to the hair; and (4) rinsing the foam from the hair.

Examples

The following examples illustrate the shampoo composition described herein. The exemplified compositions can be prepared by conventional formulation and mixing techniques. Before combining with the other ingredients in the shampoo chassis, the silicone is emulsified to form a silicone emulsion.

All parts, percentages, and ratios herein are by weight unless otherwise specified. Some components may come from suppliers as dilute solutions. The amount stated reflects the weight percent of the active material, unless otherwise specified.

**Table 1: Conditioner Chassis**

| | Wt. % |
|---|---|
| Active Cetrimonium Chloride | 2.5 |

(continued)

| | Wt. % |
|---|---|
| Fragrance | 2.4 |
| Citric acid | To pH 4-4.5 |
| Benzyl alcohol | 0.4 |
| Kathon™ CG[1] | 0.03 |
| Water | Q.S. |
| 1. Kathon™CG, Methyl chloro isothiazolinone and Methyl isothiazolinone from Dow® | |

Conditioner Chassis was prepared by convention techniques and included adding the ingredients one by one and mixing until homogeneous or dissolved and adding heat as necessary to dissolve particular ingredients.

Silicone a, b, and c have the specific silicone structure in the examples corresponds to (Ia), reproduced below:

$$M\text{-}Y\text{-}[\text{-}(N^{+}R_2\text{-}T\text{-}N^{+}R_2)\text{-}Y\text{-}]_m\text{-}M \qquad \text{(Ia)}$$

with the following values:
m is about 2,

M represents a terminal group, comprising terminal ester groups selected from

-OC(O)-Z

wherein Z is a monovalent organic residues having about 11 carbon atoms (lauric ester).

Y is a combination of groups of the formula:

—K—S—K— and -A-E-A'-or-A'-E-A-,

with S=

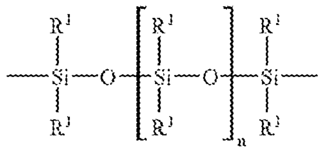

wherein $R^1$ = methyl;
for Silicone a, n =140 to 150 for an average chain length of 145;
for Silicone b, n =165 to 175 for an average chain length of 170;
for Silicone c, n = 105 to 115 for an average chain length of 115;
K is a bivalent straight chain, substituted with—OH,
A and A' each are acetic ester groups -OC(O)-$CH_2$- or -$CH_2$-C(O)O-
E is a polyethylene oxide group of the general formula:

$$\text{-}[CH_2CH_2O]_q\text{-}[CH_2CH(CH_3)O]_r\text{-}[CH_2CH(C_2H_5)O]_s\text{-}$$

wherein q=2, r=0, and s=0
T is a divalent organic group having about 6 carbon atoms.

$R_2$= methyl

Silicone a, b, and c used in the nanoemulsions and conditioner compositions described hereafter are described in Table 2, below.

**Table 2: Silicone Properties**

| | Average Number of Siloxane Repeating Units (D Units) | Molar ratio of silicone : alkylene oxide blocks | Neat Silicone Viscosity (cP) | Nitrogen Content (mmol N/g silicone polymer) |
|---|---|---|---|---|
| Silicone a | D145 | 9:1 | 10,100 | 0.16 |
| Silicone b | D170 | 9:1 | 6,400 | 0.19 |
| Silicone c | D110 | 9:1 | 15,400 | 0.24 |

Nanoemulsion A in Table 3, below, was made as follows: DI water and lactic acid were mixed to form an aqueous phase. Separately, the silicone and Tergitol™ 15-S-5 were pre-mixed and this pre-mix was slowly added to the aqueous phase.

Nanoemulsions B, C, X, and Y in Table 3, below, were made as follows: The silicone and Tergitol™ 15-S-5 were mixed. Mixing continued while the DI water was added dropwise. Finally, 0.07 g lactic acid was added.

**Table 3: Silicone Nanoemulsions**

| | Nanoemulsion | | | | |
|---|---|---|---|---|---|
| | A (wt. %) | B (wt. %) | C (wt. %) | X (wt. %) | Y (wt. %) |
| Silicone a | 20 | | | | |
| Silicone b | | 20 | | | |
| Silicone c | | | 20 | | |
| Silicone Quatemium-18 | | | | 20 | 20 |
| Tergitol™ 15-S-5[1] | 10 | 10 | 10 | 10 | 22 |
| DI water | 69.3 | 69.3 | 69.3 | 69.3 | 57.3 |
| Lactic acid | 0.7 | 0.7 | 0.7 | 0.7 | 0.7 |
| Appearance | Transparent to slightly opaque | Transparent to slightly opaque | Transparent to slightly opaque | Milky white | Milky white |
| Phase stability | Single phase | Single phase | Single phase | Single phase | Single phase |
| 1. Tergitol™ 15-S-5, C11-15 Pareth-5. Commercially available from Dow®. | | | | | |

Nanoemulsions A, B, and C are transparent to slightly opaque and Nanoemulsions X and Y are milky white. Nanoemulsions A, B, and C may be preferred, since the transparent formulation indicates that the silicone was able to be dispersed into a nanoemulsion and are more likely to be stable when they are formulated into conditioner compositions.

The appearance and phase stability was determined as follows. The example was put in a clear, glass jar. The cap was screwed on the jar, finger-tight. The example was stored at ambient temperature (20-25 °C), away from direct sunlight, for 7 days. Then the example was visually inspected to determine the appearance and phase stability. The example was considered clear if by visual detection if there are no visible particulates and it allows light to pass through so that objects behind can be distinctly seen, similar to water. On the other hand, the example was cloudy if by visual detection the example appeared to have visible material in suspension. As used herein, "visual detection" means that a human viewer can visually discern the quality of the example with the unaided eye (excepting standard corrective lenses adapted to compensate for near-sightedness, farsightedness, or stigmatism, or other corrected vision) in lighting at least equal to the illumination of a standard 100 watt incandescent white light bulb at a distance of 1 meter.

Examples 1-4, see Table 4, were made by combining, until homogeneous, 6 g of Conditioner Chassis (*see* Table 1: Conditioner Chassis) with 4 g silicone Nanoemulsion (*see* Table 3: Silicone Nanoemulsions), resulting in about 8% active silicone in the formula. The appearance and phase stability were evaluated as discussed herein.

**Table 4: Conditioner Compositions**

| | Ex. 1 | Ex. 2 | Ex. 3 | Ex. 4 |
|---|---|---|---|---|
| Conditioner Chassis | 6 g | 6 g | 6 g | 6 g |
| Nanoemulsion A | 4 g | | | |
| Nanoemulsion B | | 4 g | | |
| Nanoemulsion X | | | 4 g | |
| Nanoemulsion Y | | | | 4 g |
| Conditioner composition liquid phase viscosity | < 50 cps | < 50 cps | < 50 cps | < 50 cps |
| Appearance | Transparent | Transparent | Turbid | Turbid |
| Phase stability | Single phase | Single phase | Phase separation | Phase separation |

Examples 1 and 2, which had nanoemulsions containing silicone with an average chain length of 145 siloxane units and 170 siloxane units, respectively, did not change their appearance and were transparent and single phase after storage at room temperature for seven days. Comparative Examples 3 and 4, appeared turbid and had phase separation and may not be desirable to consumers after storage at room temperature for seven days.

The bulk conditioner samples for Table 5, below, were made by combining, until homogeneous, 60% of Conditioner Chassis (*see* Table 1: Conditioner Chassis) with 40% silicone emulsion, resulting in about 8% active silicone in the formula. Once the bulk was homogenous, 95 g of it was added to 5 g of propellant (A46 or HFO) resulting in about 5% propellant aerosol. The samples were then placed at 40 °C and imaged at various time points to track for phase stability and physical appearance changes.

**Table 5: Stability in Conditioner Chassis**

| | **Initial** | | | **8 Weeks at 40 °C** | | |
|---|---|---|---|---|---|---|
| | **No Propellant (bulk)** | **With A46** | **With HFO** | **No Propellant (bulk)** | **With A46** | **With HFO** |
| **Conditioner Chassis with Nanoemulsion C** | Clear with blue tint | Clear with blue tint | Clear with blue tint | Clear with blue tint with small clear layer on top | Clear with blue tint | Clear with blue tint with small clear layer on top |
| **Conditioner Chassis with Silsoft 253 emulsion[1]** | Clear & yellow | Clear & yellow | Clear & yellow | Turbid & Rust colored | Clear & rust colored | Turbid & Rust colored |
| **Conditioner Chassis with Silsoft Q emulsion[2]** | Clear & colorless | Clear & colorless | Clear & colorless | Clear & rust colored | Clear & rust colored with small clear layer on top | Turbid & milky colored |
| 1. Silsoft 253 is an amine functional silicone microemulsion with INCI name Amodimethicone (and) C11-15 Pareth-7 (and) Laureth-9 (and) Glycerin (and) Trideceth-12. The microemulsion is a colorless liquid and contains 20% actives with an average droplet size of less than 20 nanometers. Silsoft 253 has a neat silicone viscosity of 50 cP at 25 °C. Commercially available silicone available from Momentive Performance Materials Inc.<br>2. Silsoft Q is a cationic silicone terpolymer with INCI name water (and) Silicone Quaternium-18 (and) Trideceth-6 (and) Trideceth-12. Silsoft Q is a 20% active, microemulsion of a quaternized silicone terpolymer ("silicone quat") with a clear to slightly hazy appearance. Silsoft Q has a neat silicone viscosity of 150-200 mPas at 25 °C. Commercially available silicone from Momentive Performance Materials Inc. | | | | | | |

The comparative examples that contained commercially available amino functional silicones or silicone quats turn rust colored over time with and without propellant. This color change may not be desirable to consumers because

it can signal that something is wrong with the product and it is ineffective. In addition, a rust color may not be consumer preferred for shampoo products since it appears dark and dirty.

However, the examples with the Nanoemulsion C is color stable both with and without propellant and may be preferred by consumers. Many of these examples appeared phase stable. Others had a small clear layer on top, which may indicate that they separated slightly. However, it is hypothesized that this slight separation is not significant and does impact product performance. Thus, the stability of the example with the Nanoemulsions C is acceptable to consumers.

## Test Methods

### Foam Density & Foam Volume

Foam density is measured by placing a 30 mL density cup onto a mass balance, tarring the mass of the density cup and then dispensing product from the aerosol container into the density cup until the volume of the foam is above the rim of the vessel. The foam is made level with the top of the density cup by scraping a spatula across it within 10 seconds of dispensing the foam above the rim of the vessel. The resulting mass of the 100 ml of foam is then divided by the volume (100) to determine the foam density in units of g/ml.

Foam volume is measured by placing a weigh boat onto a mass balance, tarring the mass of the weigh boat and then dispensing the desired amount of product from the aerosol container. The grams of foam dispensed is determined and then divided by the density of foam as determined from the Foam Density methodology to reach a volume of foam in ml or $cm^3$.

### Foam Rheology Method (Yield Point)

Foam shampoo is applied to the AR1000 rheometer for foam oscillation stress sweep. 60mm smooth acrylic plate is utilized for shear stress measurement. Measurement is made at 25C. The plate head is lowered to 1200 microns and excess foam is removed with a spatula so that drag does not occur during measurement. The measurement gap height is then lowered 1000 microns. Sweep occurs from 0.1 to 400Pa. Data is analyzed via TA Rheology Advantage Data Analysis software. Yield point is determined at the point at which the oscillatory shear stress begins to deviate from its tangent. The yield point measurements are reported in Pa units.

### Kruss Lather Analyzer (Bubble Size)

The commercially available Kruss lather analyzer DFA100, supplied from Kruss, is used to analyze the foam shampoo for the initial Sauter mean radius $R_{32}$ (bubble size). Shampoo foam is dispensed into the CY4571 column containing a prism. An internal stopper is placed into the column approximately 100ml from the top of the chamber. The camera height is set to 244mm and camera position is placed in the 3 slot. Structure foaming is captured at 2 frames per second for 120 seconds. Data analysis is performed on the Kruss Advance 1.5.1.0 software application version.

### Silicone Deposition (ppm) Test Method:

Hair samples treated with different products are submitted as balls of hair with an average sample size of 0.1 g. These hair samples are then digested using a single reaction chamber microwave digestion system (Milestone Inc., Shelton, CT) using a 6:1 HNO3:H2O2 mixture and an aliquot of methyl isobutyl ketone (MIBK) in Teflon digestion vessels. A gentle digestion program with a ramp to 95°C and a manual vent after cooling below 30°C is used to facilitate retention of silicon. After dilution to volume, the samples are run against an inorganic silicon calibration curve produced on an Optima 8300 ICP-OES system (Perkin Elmer, Waltham, Massachusetts) run in the axial mode. The silicon values determined are converted to a concentration of silicone polymer-equivalents deposited on the hair sample using the theoretical silicon concentration of the polymer provided by the manufacturer. An untreated hair sample is analyzed to determine the background concentration of silicon to allow correction if needed. Another untreated hair sample is spiked with a known amount of polymer and analyzed to ensure recovery of the polymer and verify the analysis.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A concentrated conditioner composition, wherein the concentrated conditioner composition comprises:

a. from 0.5% to 25% of one or more silicones, by weight of the concentrated conditioner composition, wherein the average particle size of the one or more silicones is from 1 nm to 100 nm as measured according to dynamic light scattering as disclosed herein, and wherein at least one or more silicones is a polyorganosiloxane compound comprising:

one or more quaternary ammonium groups;
silicone blocks comprising between 105 and 180 siloxane units, preferably between 130 and 150 siloxane units;
at least one polyalkylene oxide structural unit;
at least one terminal ester group;
wherein at least one of the one or more silicones is a polyorganosiloxane has the following structure:

$$M\text{-}Y\text{-}[\text{-}(N^{+}R_2\text{-}T\text{-}N^{+}R_2)\text{-}Y\text{-}]_m\text{-}[\text{-}(NR^2\text{-}A\text{-}E\text{-}A'\text{-}NR^2)\text{-}Y\text{-}]_k\text{-}M$$

wherein:

m is 1 to 10;
k is 0;
M represents a terminal group, comprising terminal ester groups selected from

$$\text{-OC(O)-Z}$$

wherein Z is selected from monovalent organic residues having up to 20 carbon atoms;
A and A' each are independently from each other selected from a single bond or a divalent organic group having up to 10 carbon atoms and one or more hetero atoms; and
E is a polyalkylene oxide group of the general formula:

$$\text{-}[CH_2CH_2O]_q$$

wherein q=1 to 10;
$R^2$ is selected from hydrogen or R,
R is selected from monovalent organic groups having up to 22 carbon atoms; and wherein the free valencies at the nitrogen atoms are bound to carbon atoms,
Y is a group of the formula:

—K—S—K— and -A-E-A'-or-A'-E-A-,

with S=

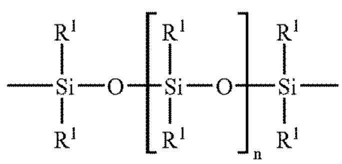

wherein R1 = $C_1$-$C_{22}$-alkyl, $C_1$-$C_{22}$-fluoralkyl or aryl; n=105 to 180, and these can be identical or different if several S Groups are present in the polyorganosiloxane compound;
K is a bivalent or trivalent straight chain, cyclic and/or branched $C_2$-$C_{40}$ hydrocarbon residue which is optionally interrupted by—O—,—NH—, trivalent N, -$NR^1$-,-C(O)-, -C(S)-, and optionally substituted with—OH, wherein $R^1$ is defined as above,
T is selected from a divalent organic group having up to 20 carbon atoms;
wherein the polyorganosiloxane compound comprises a nitrogen content from 0.13 to 0.27 mmol N/g

polymer;
wherein the one or more silicones is in the form of a nanoemulsion emulsified by a nonionic surfactant, and wherein the concentrated conditioner composition comprises from 1% to 15% of the nonionic emulsifier, by weight of the concentrated conditioner composition;
wherein the emulsifier is a condensation product of an aliphatic alcohol having from 8 to 18 carbons, in either straight chain or branched chain configuration, with from 2 to 35 moles of ethylene oxide;

b. from 60% to 90% water, by weight of the concentrated conditioner composition; wherein the concentrated conditioner composition has a liquid phase viscosity of from 1 mPa.s to 15,000 mPa.s as measured by the Cone/Plate Viscosity Measurement as disclosed herein.

**2.** The composition according to claim 1, wherein the viscosity of the neat silicone polymer is from 2,000 to 50,000 mPa.s, preferably from 8,000 to 50,000 mPa.s, more preferably from 8,000 to 30,000 mPa.s as measured by the Cone/Plate Viscosity Measurement as disclosed herein.

**3.** The composition of any of the preceding claims, wherein the average particle size of the one or more silicones is from 10 nm to 90 nm with a polydispersity index of less than 0.2, preferably the average particle size of the one or more silicones is from 40 nm to 80 nm with a polydispersity index of less than 0.2 as measured according to dynamic light scattering as disclosed herein.

**4.** The composition of any of the preceding claims, wherein the concentrated conditioner composition comprises from 1% to 20% of the one or more silicones, by weight of the concentrated conditioner composition, preferably from 2% to 18% of the one or more silicones, by weight of the concentrated conditioner composition, more preferably from 2% to 10% of the one or more silicones, by weight of the concentrated conditioner composition.

**5.** The composition of any of the preceding claims, wherein the concentrated conditioner composition is for rinse-off use.

**6.** A method of conditioning the hair comprising:

a. providing a conditioner composition in a foam dispenser;
b. dispensing the conditioner composition according to any of the preceding claims from the foam dispenser as a dosage of foam;
c. applying the foam to the hair; and
d. rinsing the foam from the hair.

**7.** The method of Claim 6, wherein the foam dispenser is a mechanical foam dispenser or an aerosol foam dispenser.

## Patentansprüche

**1.** Konzentrierte Conditioner-Zusammensetzung, wobei die konzentrierte Conditioner-Zusammensetzung umfasst:

a. von zu 0,5 % bis 25 % ein oder mehrere Silikone, bezogen auf das Gewicht der konzentrierten Conditioner-Zusammensetzung, wobei die durchschnittliche Teilchengröße des einen oder der mehreren Silikone von 1 nm bis 100 nm beträgt, wie gemäß einer dynamischen Lichtstreuung gemessen, wie hierin offenbart, und wobei wenigstens ein oder mehrere Silikone eine Polyorganosiloxanverbindung ist, umfassend:

eine oder mehrere quartäre Ammoniumgruppen;
Silikonblöcke, umfassend zwischen 105 und 180 Siloxaneinheiten, vorzugsweise zwischen 130 und 150 Siloxaneinheiten;
wenigstens eine Polyalkylenoxidstruktureinheit;
wenigstens eine endständige Estergrupp;
wobei mindestens eines des einen oder der mehreren Silikone ein Polyorganosiloxan ist, das die folgende Struktur aufweist:

$$M\text{-}Y\text{-}[\text{-}(N^+R_2\text{-}T\text{-}N^+R_2)\text{-}Y\text{-}]_m\text{-}[\text{-}(NR^2\text{-}A\text{-}E\text{-}A'\text{-}NR^2)\text{-}Y\text{-}]_k\text{-}M$$

wobei:

m 1 bis 10 ist;
k 0 ist;
M eine endständige Gruppe darstellt, umfassend endständige Estergruppen, ausgewählt aus

-OC(O)-Z

wobei Z aus einwertigen organischen Rückständen, die bis zu 20 Kohlenstoffatome aufweisen, ausgewählt ist;
A und A' jeweils unabhängig voneinander aus einer Einfachbindung oder einer zweiwertigen organischen Gruppe ausgewählt sind, die bis zu 10 Kohlenstoffatome und ein oder mehrere Heteroatome aufweist; und
E eine Polyalkylenoxidgruppe der allgemeinen Formel ist:

$-[CH_2CH_2O]_q$

wobei q = 1 bis 10;
$R^2$ aus Wasserstoff oder R ausgewählt ist,
R aus einwertigen organischen Gruppen ausgewählt ist, die bis zu 22 Kohlenstoffatome aufweisen; und wobei die freien Valenzen an den Stickstoffatomen an Kohlenstoffatome gebunden sind,
Y eine Gruppe der folgenden Formel ist:

-K-S-K- und -A-E-A'-oder-A'-E-A-,

mit S =

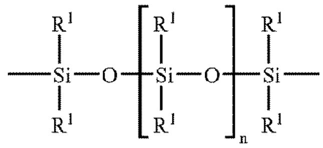

wobei R1 = $C_1$-$C_{22}$-Alkyl, $C_1$-$C_{22}$-Fluoralkyl oder -Aryl; n = 105 bis 180, und diese können identisch oder unterschiedlich sein, wenn mehrere S-Gruppen in der Polyorganosiloxanverbindung vorhanden sind;
K eine bivalente oder dreiwertige gerade Kette, cyclischer und/oder verzweigter $C_2$-$C_{40}$-Kohlenwasserstoffrückstand ist, der wahlweise durch -O-, -NH-, dreiwertiges N, $-NR^1-$, -C(O)-, -C(S)- unterbrochen ist, und wahlweise mit -OH substituiert ist, wobei $R^1$ wie oben definiert ist,
T aus einer zweiwertigen organischen Gruppe ausgewählt ist, die bis zu 20 Kohlenstoffatome aufweist;
wobei die Polyorganosiloxanverbindung einen Stickstoffgehalt von 0,13 bis 0,27 mmol N/g Polymer umfasst;
wobei das eine oder die mehreren Silikone in der Form einer Nanoemulsion vorliegen, die durch ein nichtionisches Tensid emulgiert ist, und wobei die konzentrierte Conditioner-Zusammensetzung von 1 % bis 15 % den nichtionischen Emulgator, bezogen auf das Gewicht der konzentrierten Conditioner-Zusammensetzung, umfasst;
wobei der Emulgator ein Kondensationsprodukt eines aliphatischen Alkohols, der 8 bis 18 Kohlenstoffe aufweist, entweder in geradkettiger oder verzweigtkettiger Konfiguration, mit von 2 bis 35 Mol Ethylenoxid ist;

b. von zu 60 % bis 90 %, bezogen auf das Gewicht der konzentrierten Conditioner-Zusammensetzung;
wobei die konzentrierte Conditioner-Zusammensetzung eine Flüssigphasenviskosität von 1 mPa.s bis 15.000 mPa.s, gemessen durch die Kegel/Platte-Viskositätsmessung, wie hierin offenbart, aufweist.

**2.** Zusammensetzung nach Anspruch 1, wobei die Viskosität des unverdünnten Silikonpolymers von 2.000 bis 50.000 mPa.s, vorzugsweise von 8.000 bis 50.000 mPa.s, mehr bevorzugt von 8.000 bis 30.000 mPa.s, gemessen durch die Kegel/Platte-Viskositätsmessung, wie hierin offenbart, beträgt.

**3.** Zusammensetzung nach einem der vorstehenden Ansprüche, wobei die durchschnittliche Teilchengröße des einen oder der mehreren Silikone von 10 nm bis 90 nm mit einem Polydispersitätsindex von weniger als 0,2 beträgt,

vorzugsweise die durchschnittliche Teilchengröße des einen oder der mehreren Silikone von 40 nm bis 80 nm mit einem Polydispersitätsindex von weniger als 0,2, wie nach der dynamischen Lichtstreuung gemessen, wie hierin offenbart, beträgt.

**4.** Zusammensetzung nach einem der vorstehenden Ansprüche, wobei die konzentrierte Conditioner-Zusammensetzung von 1 % bis 20 % das eine oder die mehreren Silikone, bezogen auf das Gewicht der konzentrierten Conditioner-Zusammensetzung, vorzugsweise von 2 % bis 18 % das eine oder die mehreren Silikone, bezogen auf Gewicht der konzentrierten Conditioner-Zusammensetzung, mehr bevorzugt von 2 % bis 10 % das eine oder die mehreren Silikone, bezogen auf das Gewicht der konzentrierten Conditioner-Zusammensetzung umfasst.

**5.** Zusammensetzung nach einem der vorstehenden Ansprüche, wobei die konzentrierte Conditioner-Zusammensetzung für eine Abspülverwendung dient.

**6.** Verfahren zum Konditionieren des Haars, umfassend:

a. Bereitstellen einer Conditioner-Zusammensetzung in einem Schaumspender;
b. Abgeben der Conditioner-Zusammensetzung nach einem der vorstehenden Ansprüche aus dem Schaumspender als eine Dosis von Schaum;
c. Aufbringen des Schaums auf das Haar; und
d. Spülen des Schaums aus dem Haar.

**7.** Verfahren nach Anspruch 6, wobei der Schaumspender ein mechanischer Schaumspender oder ein Aerosolschaumspender ist.

## Revendications

**1.** Composition de conditionneur concentrée, dans laquelle la composition de conditionneur concentrée comprend :

a. de 0,5 à 25 % d'une ou plusieurs silicones, en poids de la composition de conditionneur concentrée, dans laquelle la taille moyenne de particules de la ou des silicones va de 1 nm à 100 nm telle que mesurée selon la dispersion dynamique de lumière telle que décrite ici, et dans laquelle au moins une ou plusieurs silicones sont des composés polyorganosiloxane comprenant :

un ou plusieurs groupes d'ammonium quaternaire ;
des blocs de silicone comprenant entre 105 et 180 motifs siloxane, de préférence entre 130 et 150 motifs siloxane ;
au moins un motif structural poly(oxyde d'alkylène) ;
au moins un groupe ester terminal ;
dans laquelle au moins l'une des unes ou plusieurs silicones est un polyorganosiloxane qui a la structure suivante :

$$M\text{-}Y\text{-}[\text{-}(N^+R_2\text{-}T\text{-}N^+R_2)\text{-}Y\text{-}]_m{=}[\text{-}(NR^2\text{-}A\text{-}E\text{-}A\text{-}NR^2)\text{-}Y\text{-}]_k\text{-}M$$

dans lequel :

m va de 1 à 10 ;
k vaut 0 ;
M représente un groupe terminal, comprenant des groupes ester terminaux choisis parmi

$$\text{-}OC(O)\text{-}Z$$

dans laquelle Z est choisi parmi des résidus organiques monovalents ayant jusqu'à 20 atomes de carbone ;
A et A' sont chacun indépendamment l'un de l'autre choisis parmi une liaison simple ou un groupe organique divalent ayant jusqu'à 10 atomes de carbone et un ou plusieurs hétéroatomes ; et
E est un groupe poly(oxyde d'alkylène) de formule générale :

$-[CH_2CH_2O]_q$

dans laquelle q = 1 à 10 ;
$R^2$ est choisi parmi hydrogène ou R,
R est choisi parmi des groupes organiques monovalents ayant jusqu'à 22 atomes de carbone ; et dans laquelle les valences libres au niveau des atomes d'azote sont liées à des atomes de carbone,
Y est un groupe de formule :

-K-S-K- et -A-E-A'- ou -A'-E-A-,

avec S =

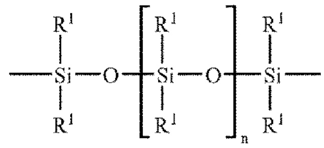

dans laquelle R1 = alkyle $C_1$-$C_{22}$, fluoroalkyle ou aryle $C_1$-$C_{22}$, n=105 à 180, et ceux-ci peuvent être identiques ou différents si plusieurs groupes S sont présents dans le composé polyorganosiloxane,
K est une chaîne linéaire bivalente ou trivalente, un résidu hydrocarbure cyclique et/ou ramifié $C_2$-$C_{40}$ qui est éventuellement interrompu par-O-, -NH-, N trivalent, $-NR^1-$, -C(O)-, -C(S)-, et éventuellement substitué par -OH, dans laquelle $R^1$ est défini tel que ci-dessus,
T est choisi parmi un groupe organique divalent ayant 20 atomes de carbone maximum ;
dans laquelle le composé polyorganosiloxane comprend une teneur en azote allant de 0,13 à 0,27 mmol N/g polymère ;
dans laquelle la ou les silicones sont sous la forme d'une nanoémulsion émulsifiée par un agent tensioactif non ionique, et dans laquelle la composition de conditionneur concentrée comprend de 1 % à 15 % de l'émulsifiant non ionique, en poids de la composition de conditionneur concentrée ;
dans laquelle l'émulsifiant est un produit de condensation d'un alcool aliphatique ayant de 8 à 18 carbones, dans une configuration à chaîne linéaire ou ramifiée, avec de 2 à 35 moles d'oxyde d'éthylène ;

b. de 60 à 90 % d'eau, en poids de la composition de conditionneur concentrée ;
dans laquelle la composition de conditionneur concentrée a une viscosité de phase liquide allant de 1 mPa.s à 15 000 mPa.s telle que mesurée par la mesure de viscosité de cone/plaque telle que décrite ici.

**2.** Composition selon la revendication 1, dans laquelle la viscosité du polymère de silicone pur va de 2 000 à 50 000 mPa.s, de préférence de 8 000 à 50 000 mPa.s, plus préférablement de 8 000 à 30 000 mPa.s telle que mesurée par la mesure de viscosité de cone/plaque telle que décrite ici.

**3.** Composition selon l'une quelconque des revendications précédentes, dans laquelle la taille moyenne de particules de la ou des silicones va de 10 à 90 nm avec un indice de polydispersité inférieur à 0,2, de préférence la taille moyenne de particules de la ou des silicones va de 40 nm à 80 nm avec un indice de polydispersité inférieur à 0,2 tel que mesuré selon la dispersion dynamique de lumière telle que décrite ici.

**4.** Composition selon l'une quelconque des revendications précédentes, dans laquelle la composition de conditionneur concentrée comprend de 1 à 20 % de la ou des silicones, en poids de la composition de conditionneur concentrée, de préférence de 2 à 18 % de la ou des silicones, en poids de la composition de conditionneur concentrée, plus préférablement de 2 à 10 % de la ou des silicones, en poids de la composition de conditionneur concentrée.

**5.** Composition selon l'une quelconque des revendications précédentes, dans laquelle la composition de conditionneur concentrée est destinée à être utilisée par rinçage.

**6.** Procédé de conditionnement de cheveux comprenant :

a. la fourniture d'une composition de conditionneur dans un distributeur de mousse ;
b. la distribution de la composition de conditionneur selon l'une quelconque des revendications précédentes à

partir du distributeur de mousse en guise de dosage de mousse ;
c. l'application de la mousse sur les cheveux ; et
d. le rinçage de la mousse des cheveux.

**7.** Procédé selon la revendication 6, dans lequel le distributeur de mousse est un distributeur de mousse mécanique ou un distributeur de mousse aérosol.

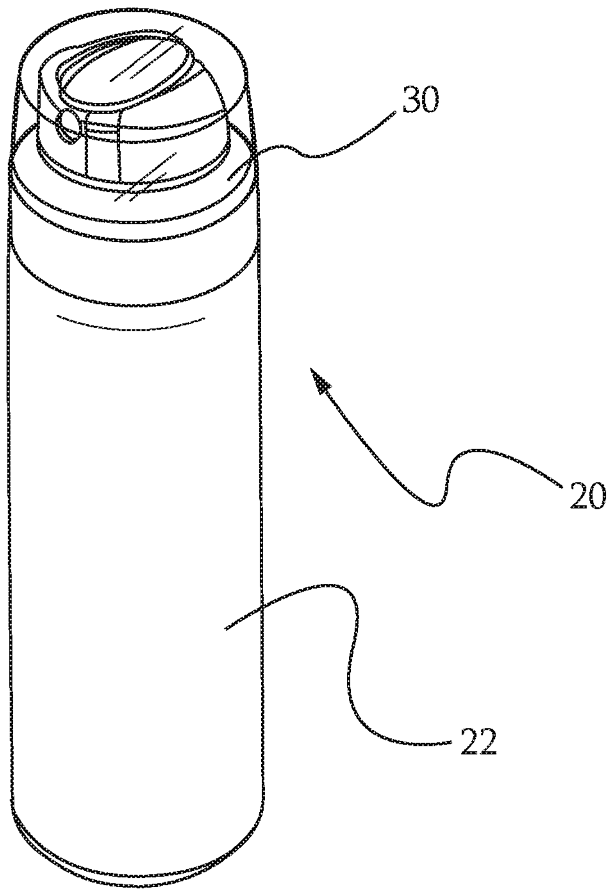


Fig. 1

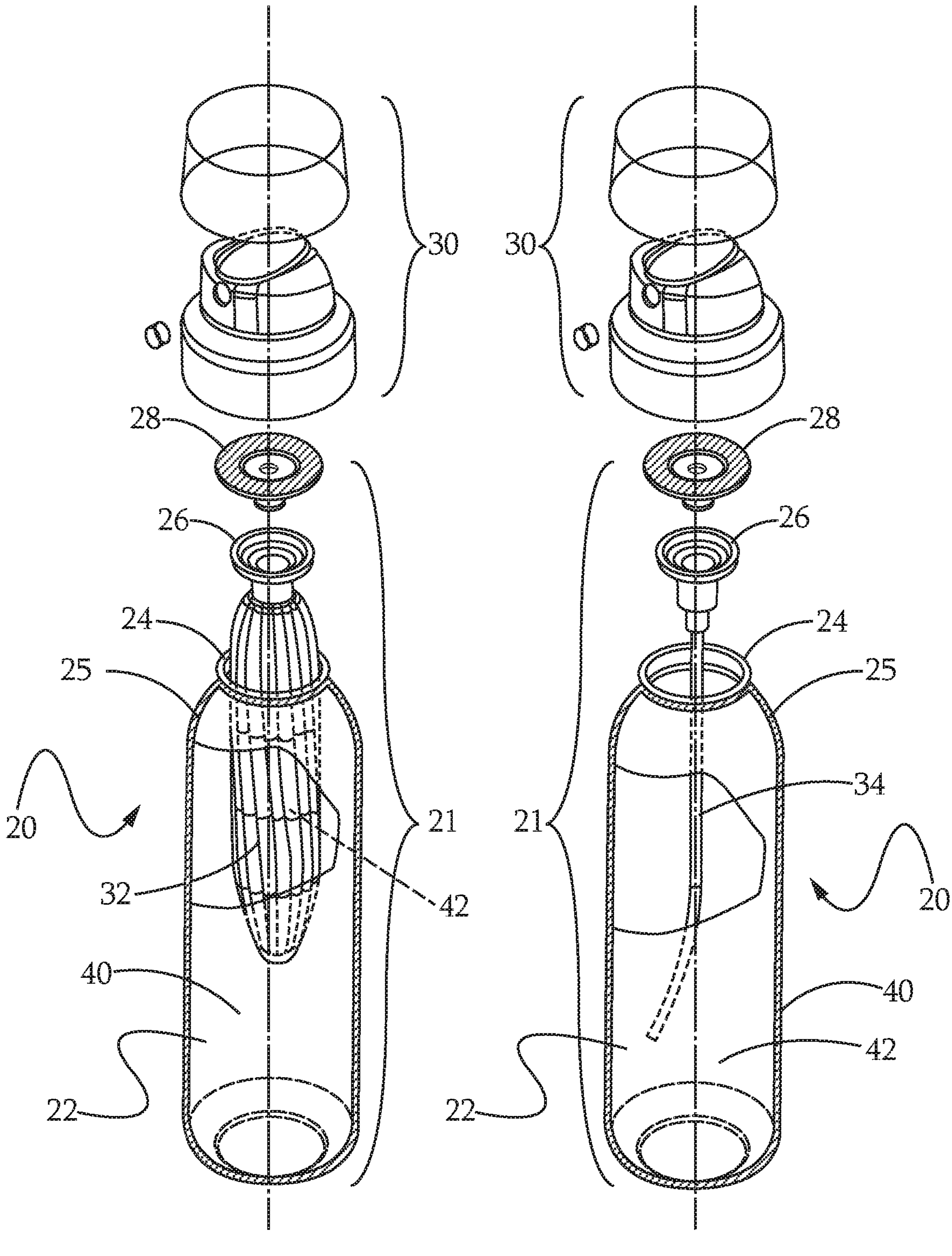


Fig. 2A

Fig. 2B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150359725 A1
- US 20150359726 A1
- US 2015093420 A
- WO 2013148629 A
- WO 2013148778 A
- EP 0283165 B1
- WO 2004078903 A
- WO 2004078901 A
- WO 2005078063 A

### Non-patent literature cited in the description

- International Cosmetic Ingredient Dictionary. 1993
- CTFA Cosmetic Ingredient Handbook. 1992
- CTFA Cosmetic Ingredient Handbook. The Cosmetic, Toiletries, and Fragrance Association, Inc, 1988
- International Buyers Guide. CFTA Publications, 1992
- Chemicals Buyers Directory. Schnell Publishing Co, 1993